# EUROPÄISCHE PATENTSCHRIFT

(11) **EP 0 635 303 B1**
(45) Veröffentlichungstag und Bekanntmachung des Hinweises auf die Patenterteilung: **03.03.1999**
(21) Anmeldenummer: 94110877.1
(22) Anmeldetag: 13.07.1994
(51) Int. Cl.: B01J 31/02, C07C 205/12, C07B 37/04

(54) **Katalysator für nukleophile aromatische Substitutionen**
Catalyst for nucleophilic aromatic substitutions
Catalyseur pour des substitutions aromatiques nucléophiles

(30) Priorität: 21.07.1993 DE 4324366
(43) Veröffentlichungstag der Anmeldung: 25.01.1995
(73) Patentinhaber: Clariant GmbH, 65929 Frankfurt am Main (DE)
(72) Erfinder: Schach, Dr. Thomas, D-64579 Gernsheim (DE); Papenfuhs, Dr. Theodor, D-60433 Frankfurt am Main (DE)

(56) Entgegenhaltungen:
- EP-A- 0 523 671
- WO-A-92/00270

## Beschreibung

Gegenstand der vorliegenden Erfindung ist ein neues Katalysatorsystem, das eine Vielzahl von nukleophilen Substitutionen an Aromaten beschleunigt oder überhaupt erst ermöglicht.

Nucleophile Substitutionen spielen eine bedeutende Rolle in der Synthese von substituierten Aromaten. Zur Durchführung dieser Reaktionen werden vergleichsweise hohe Reaktionstemperaturen benötigt, oft zwischen 200 und 320°C, wodurch zum Teil erhebliche Anteile an Zersetzungsprodukten entstehen. Im allgemeinen kann auf ein Lösungsmittel nicht verzichtet werden, so daß die Raum/Zeitausbeuten im Vergleich zu lösungsmittelfreien Verfahren deutlich niedriger liegen.

Als Alternative können herkömmlliche Phasentransferkatalysatoren verwendet werden, durch die sich einige der oben beschriebenen Nachteile verbessern lassen. Andere Probleme, wie beispielsweise eine schlechte Rührbarkeit der Reaktionssuspension bei lösungsmittelfreien Verfahren bleiben weiter bestehen. Bislang wurden als Phasentransferkatalysatoren quartäre Alkylammonium- oder Alkylphosphoniumsalze US-A-4,287,374, Pyridiniumsalze (WO 87/04149) oder Kronenether verwendet, die zum Teil nur geringe Reaktivitäten zeigen oder unter den benötigten Reaktionstemperaturen nur mäßig stabil sind.

EP-A-0 523 671 und WO-A-9 200 270 beschreiben Verfahren zur Herstellung von Chlorfluornitrobenzolen, indem man Dichloronitrobenzole mit Alkalimetallfluoriden in Gegenwart eines quatären Ammonium- und/oder Phosphoniumsalzes, Kronethers und/oder Polyethylenglycoldimethylethers als Katalysator in Anwesenheit eines Lösungsmittels umsetzt.

In Anbetracht dieser Einschränkungen und Nachteile bestand ein großes Bedürfnis nach einem verbesserten Katalysatorsystem, durch das die den bekannten Verfahren innewohnenden Nachteile vermieden und gute bis sehr gute Ausbeuten, niedrigere Reaktionstemperaturen und verkürzte Reaktionszeiten ermöglicht und geringere Mengen an polymeren Zersetzungsprodukten erhalten werden. Insbesondere der Bewältigung von Rührproblemen und Aufarbeitungsproblemen in lösungsmittelfreien Verfahren und in Verfahren mit nur sehr geringen Lösungsmittelmengen wurde eine besondere Bedeutung beigemessen.

Es wurde gefunden, daß eine Mischung aus einer quartären Ammoniumverbindung, die mindestens einen Alkoxypolyoxyalkyl-Rest enthält, einem quartären Ammonium- oder Phosphoniumsalz und/oder einem Polyether überraschenderweise die vorstehend genannten Forderungen erfüllt.

Gegenstand der vorliegenden Erfindung ist ein Katalysator für nukleophile Substitutionen, bestehend im wesentlichen aus einer Mischung aus
a) einer oder mehreren quartären Ammoniumverbindung(en) der Formel (1) 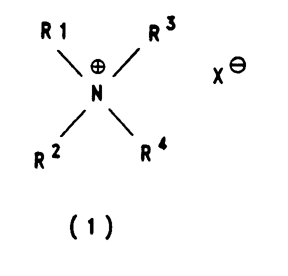 worin
   - R¹, R² und R³: gleich oder verschieden sind und
   einen linearen oder verzweigten Rest der Formel -(CₘH₂ₘO)ₚR⁵ bedeuten, worin R⁵ für Wasserstoff oder einen linearen oder verzweigten Alkylrest mit 1 bis 16, vorzugsweise 1 bis 8, Kohlenstoffatomen steht, m eine ganze Zahl von 1 bis 10, vorzugsweise 1 bis 5, und p eine Zahl von 1 bis 15, vorzugsweise 2 bis 10, bedeuten; oder
   einen linearen oder verzweigten Alkylrest mit 1 bis 30, vorzugsweise 1 bis 18, Kohlenstoffatomen; oder einen unsubstituierten Phenyl-oder Naphthylrest; oder einen substituierten Phenyl- oder Naphthylrest, wobei die Substituenten die Bedeutung Halogen, C₁-C₄-Alkyl, C₁-C₄-Alkoxy, Nitro oder Cyano haben;
   - R⁴: einen linearen oder verzweigten Rest der Formel -(CₘH₂ₘO)ₚR⁵ bedeutet; und
   - X^{⊖}: ein anorganisches Anion, vorzugsweise Fluorid, Chlorid, Bromid, SO₄²⁻/2 oder Hydrogensulfat, ist;
   und
b) einem oder mehreren quartären Ammoniumsalz(en) oder Phosphoniumsalz(en) der Formel (2) 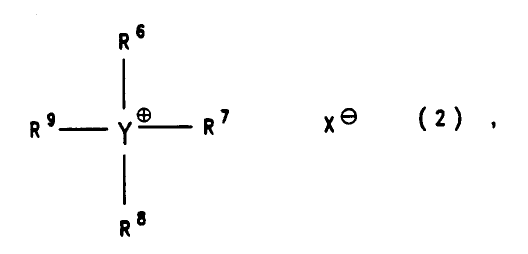 worin
   - R⁶, R⁷, R⁸ und R⁹: gleich oder verschieden sind und
   einen linearen oder verzweigten Alkylrest mit 1 bis 22, vorzugsweise 1 bis 16, Kohlenstoffatomen; oder einen unsubstituierten oder substituierten Arylrest oder einen C₁-C₄-Alkyl-arylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und
   - Y: die Bedeutung N oder P hat;
   oder aus einer Mischung aus einer oder mehreren Verbindung(en) der Formel (1) und
c) einem oder mehreren Ether(n) der Formel (3)

   R¹⁰-(O-CₓH₂ₓ)ᵣ-OR¹¹ (3),

   worin
   - R¹⁰ und R¹¹: gleich oder verschieden sind und Wasserstoff oder einen linearen oder verzweigten Alkylrest mit 1 bis 16, vorzugsweise 1 bis 8, Kohlenstoffatomen bedeuten;
   - x: eine ganze Zahl von 2 bis 6, vorzugsweise 2 bis 3, und
   - r: eine Zahl von 0 bis 20, vorzugsweise 4 bis 14, ist;
   oder einem Kronenether;
   oder aus einer Mischung der in a), b) und c) genannten Verbindungen.

Das Anion X^{⊖} kann in den Verbindungen der Formel (1) und (2) jeweils die gleiche oder verschiedene Bedeutungen haben.

Die Mischungsverhältnisse der Komponenten a) und b), a) und c), sowie a), b) und c) können in einem weiten Bereich schwanken, mit der Maßgabe, daß die Komponente a) 5 bis 95 Gew.-%, vorzugsweise 20 bis 80 Gew.-%, des gesamten Katalysators ausmacht.

In dem in der Verbindung der Formel (1) enthaltenen linearen oder verzweigten Rest der Formel -(CₘH₂ₘO)ₚR⁵ können gleiche oder unterschiedliche Alkoxy-Einheiten miteinander verknüpft sein.
Die Anzahl der in der Verbindung der Formel (1) enthaltenen linearen oder verzweigten Reste beträgt vorzugsweise 1 oder 2. Besonders bevorzugte Verbindungen der Formel (1) im Sinne der vorliegenden Erfindung sind Dimethyl-di(ethoxypolyoxypropyl)-ammoniumchlorid, Dimethyldi(ethoxypolyoxypropylmethylether)-ammoniumchlorid, Dimethyl(ethoxypolyoxypropyl)-(ethoxypolyoxypropylmethylether)-ammoniumchlorid, Dimethyl-di(ethoxypolyoxyethyl)-ammoniumchlorid, Dimethyldi(ethoxypolyoxyethylmethylether)-ammoniumchlorid, Dimethyl(ethoxypolyoxyethyl)-(ethoxypolyoxyethylmethylether)-ammoniumchlorid, jeweils mit einer mittleren Kettenlänge p von 3, weiterhin Trimethyl(ethoxypolyoxypropyl)-ammoniumchlorid und Trimethyl(ethoxypolyoxypropylmethylether)-ammoniumchlorid, jeweils mit einer mittleren Kettenlänge p von 8, oder ein Gemisch der genannten Verbindungen.

Die beschriebenen Verbindungen der Formel (1) lassen sich auf bekannte Weise (US-PS 3 123 641; US-PS 3 141 905) aus den entsprechenden Ethanolaminen herstellen, die nach Umsetzung mit Alkylenoxiden und anschließender Quaternisierung mit oder ohne gleichzeitiger Veretherung in guten Ausbeuten die gewünschten Verbindungen liefern.

Bevorzugte Verbindungen der Formel (2) im Sinne der vorliegenden Erfindung sind Octadecyltrimethylammoniumchlorid, Distearyldimethylammoniumchlorid, Tetramethylammoniumchlorid, Tetramethylammoniumbromid, Hexadecyltrimethylammoniumchlorid, Benzyltrimethylammoniumchlorid, Hexadecyltributylphosphoniumbromid, Stearyltributylphosphoniumbromid, Tetrabutylphosphoniumchlorid, Tetrabutylphosphoniumbromid und Tetraoctylphosphoniumbromid.

Bevorzugte Ether der Formel (3) im Sinne der vorliegenden Erfindung besitzen eine mittlere Molmasse zwischen 300 und 800. Besonders bevorzugt ist ein Gemisch von Polyethylenglykoldimethylethern der Kettenlängen r von 6 bis 17 und einer mittleren Molmasse von 500. Anstelle von oder in Kombination mit Polyethern der Formel (3) können auch Kronenether, beispielsweise 18-Krone-6, eingesetzt werden.

Als Ausgangsverbindungen für die erfindungsgemäß katalysierte nukleophile aromatische Substitution kommen Aromaten vom Benzol-, Naphthalin- und Pyridin-Typ in Betracht, die mindestens eine der Abgangsgruppen F⁻, Cl⁻, Br⁻, J⁻, NO₂⁻ oder SO₃⁻ und mindestens eine Elektronenakzeptor-Gruppe aus der Reihe -CF₃, -CCl₃, -CN, -NO₂, -COOH, -COCl, -SO₂Cl, -COBr, -SO₂Br, -COF, -SO₂F, SO₃H oder -SO₃-alkyl enthalten. Mehrfach halogenierte Aromaten können auch ohne eine Elektronenakzeptor-Gruppe zur Reaktion gebracht werden. Die besagten Aromaten können auch weitere Substituenten enthalten, beispielsweise Alkylreste, Aminogruppen, Hydroxygruppen oder Alkoxygruppen.

Als Nukleophile für die erfindungsgemäß katalysierte nukleophile aromatische Substitution werden Fluoride, Cyanide, Hydroxide, Alkoholate oder Aminsalze von Alkalimetallen oder einwertigen Übergangsmetallen eingesetzt. Zur Einführung der CN-Gruppe ist insbesondere CuCN geeignet. Zur Einführung von Phenolat-, SH-, SR- oder Amin-Gruppen sind oftmals bereits die jeweiligen Phenole, Mercaptane oder Amine selbst ausreichend, so daß auf den Einsatz des entsprechenden Metallsalzes verzichtet werden kann.

Bei der nukleophilen aromatischen Substitution wird der erfindungsgemäße Katalysator zweckmäßigerweise in Mengen von 1 bis 35 Gew.-%, vorzugsweise von 5 bis 25 Gew.-%, bezogen auf die aromatische Ausgangsverbindung, eingesetzt. Das molare Verhältnis von Katalysator zum Aromaten ist dabei gleich oder kleiner als 1:8, bevorzugt 1:25 bis 1:100.

Was das Mengenverhältnis der Ausgangsverbindung zum Nukleophil anbelangt, so werden zweckmäßigerweise 1,0 bis 2,5 Mol Nukleophil pro Mol Aromat eingesetzt. In Fällen, in denen ein Überschuß an Nukleophil zu Nebenreaktionen führt, kann das Nukleophil auch im Unterschuß eingesetzt werden. Der erfindungsgemäße Katalysator ist allgemein für nukleophile aromatische Substitutionen geeignet, insbesondere jedoch für Chlor-Fluoraustauschreaktionen zur Herstellung von Fluoraromaten, für Halogen-Alkoxyaustauschreaktionen oder für Brom- Cyanaustauschreaktionen.

Mehrfache Austauschreaktionen ohne Lösungsmittel waren bislang nur bedingt möglich. Der hohe Salzgehalt der Reaktionssuspension führte in der Regel zu nicht rührbaren Systemen, die selbst unter günstigsten Bedingungen nur zu geringen Umsätzen und Ausbeuten führten. Durch die Verwendung des erfindungsgemäßen Katalysators kommt es nunmehr selbst bei sehr hohen Salzgehalten in der Reaktionssuspension zu keinen Rührproblemen, so daß auch zweifache Austausch-Reaktionen meist problemlos ohne Lösungsmittel durchgeführt werden können.

Überraschenderweise wird durch die Verwendung des erfindungsgemäßen Katalysators die Viskosität der Reaktionssuspension verringert. Die gleichzeitig deutlich niedrigeren Reaktionstemperaturen im Vergleich zum Stand der Technik führen zusammen mit der guten Rührbarkeit der Reaktionssuspension letztendlich zu einer deutlichen Steigerung der Ausbeute und einer Verminderung von Nebenreaktionen. Während bislang Temperaturen von 200°C bis über 300°C für nukleophile aromatische Substitutionen erforderlich waren, liegen die Reaktionstemperaturen bei Verwendung des erfindungsgemäßen Katalysators bei 20 bis 200°C.

Der erfindungsgemäße Katalysator kann in Gegenwart oder Abwesenheit von Lösungsmitteln verwendet werden. Werden Lösungsmittel verwendet, so sind sowohl aprotische und dipolar aprotische als auch protische Lösungsmittel geeignet. Geeignete dipolar aprotische Lösungsmittel sind beispielsweise Dimethylsulfoxid, Dimethylsulfon, Sulfolan, Dimethylformamid, Dimethylacetamid, 1,3-Dimethylimidazolin-2-on, Acetonitril und Benzonitril. Geeignete aprotische Lösungsmittel ohne ausgeprägten dipolaren Charakter sind beispielsweise Benzol, Toluol, Xylol, Chlortoluole, Chlorbenzol und Dichlorbenzole. Die Verwendung von protischen Lösungsmitteln, wie beispielsweise Alkoholen, ist ebenfalls möglich. Als protische Lösungsmittel werden Methanol, Ethanol, Propanol, Butanol, i-Propanol oder Polyalkylenglykole mit Ethylen-, Propylen- oder Butyleneinheiten verwendet.

Das aprotische oder dipolar aprotische Lösungsmittel kann in beliebigen Mengen verwendet werden, bevorzugt werden allerdings geringe Mengen im Bereich von 5 bis 30 Gew.-%, bezogen auf den eingesetzten Aromaten. Bei der Verwendung von protischen Lösungsmitteln liegen die eingesetzten Mengen im Bereich von 0,1 bis 5 Gew.-%, bevorzugt 0,1 bis 2 Gew.-%, bezogen auf den eingesetzten Aromaten.

Der erfindungsgemäße Katalysator kann sowohl bei Atmosphärendruck als auch bei Überdruck- oder Unterdruck verwendet werden. Diese Eigenschaft wird beispielsweise genutzt, indem geringe Mengen eines leichtsiedenden aprotischen Lösungsmittels, das mit Wasser ein Azeotrop bildet, wie beispielsweise Benzol, Xylol, Mesitylen oder Toluol, vor Beginn der Reaktion in die Reaktionssuspension gegeben werden. Anschließend wird ein Teil des Lösungsmittels durch Anlegen eines Unterdrucks zusammen mit Wasser aus der Reaktionssuspension wieder entfernt. Durch diese Verfahrensweise lassen sich die Reaktionsgeschwindigkeit und die Ausbeute steigern und die Bildung von Nebenprodukten minimieren.

Der erfindungsgemäße Katalysator kann in Anwesenheit oder Abwesenheit von Luftsauerstoff verwendet werden, bevorzugt wird das Arbeiten unter Schutzgas, wie beispielsweise Argon oder Stickstoff.

Bei der Verwendung des erfindungsgemäßen Katalysators ist zu gewährleisten, daß während der gesamten Reaktion das Reaktionsgemisch gut durchmischt wird.

Die durch nukleophile aromatische Substitution herstellbaren Produkte spielen eine bedeutende Rolle als Zwischenprodukte im Bereich des Pflanzenschutzes und als Synthesebausteine für Pharmazeutika und Farbstoffe.

Die folgenden Beispiele dienen zur Erläuterung des erfindungsgemäßen Katalysators, ohne sich darauf zu beschränken. Unter "Polyethylenglykoldimethylether 500" wird besagter Polyether mit einer mittleren Molmasse von etwa 500 verstanden. Das in den Beispielen verwendete Trimethyl-(ethoxypolyoxypropyl)-ammoniumchlorid hat eine mittlere Kettenlänge p von 8 und wurde als 84 bis 89 gew.-%iges Produkt eingesetzt. In diesem Produkt sind noch 10 bis 13 Gew.-% freies Polypropylenglykol und bis zu 2 Gew.-% Wasser enthalten.

Das eingesetzte Dimethyl-di(ethoxypolyoxypropyl)-ammoniumchlorid hat eine mittlere Kettenlänge p von 3 und ist ein 90 bis 95 gew.-%iges Produkt, das noch 5 bis 10 Gew.-% Polypropylenglykol und etwa 0,2 Gew.-% Wasser enthält.

Wurden die beiden Katalysatoren als veretherte Verbindungen verwendet, so lagen die Polypropylenglykole ebenfalls in veretherter Form vor. Der Veretherungsgrad lag im Falle von Dimethoxy-di(ethoxypolyoxypropylmethylether)-ammoniumchlorid bei 86%.

Der zeitliche Reaktionsverlauf wurde durch gaschromatographische Analyse (GC) verfolgt und die jeweils in der Reaktionsmischung vorhandene Menge des gewünschten Produktes in Form von GC-Flächenprozenten angegeben.

### Beispiel 1: 2,4-Difluornitrobenzol

In einen 500 ml Planschliffkolben mit Destillationsbrücke und Impellerrührer wurden bei 100°C in die Schmelze von 120 g (0,62 mol) 2,4-Dichlornitrobenzol 72,0 g (1,24 mol) Kaliumfluorid, 14,0 g (0,027 mol) Dimethyl-di-(ethoxypolyoxypropyl)-ammoniumchlorid und 7,0 g (0,014 mol) Polyethylenglykoldimethylether 500 eingetragen. Die Temperatur wurde auf 120°C erhöht und die Reaktionssuspension 28 Stunden bei dieser Temperatur gerührt.
Entstandene Menge an 2,4-Difluornitrobenzol:
- nach 6 Stunden:: 11 GC-Flächen-%
- nach 28 Stunden:: 56 GC-Flächen-%.

### Beispiel 2: 2-Fluornitrobenzol

In einen 2-Liter Planschliffkolben mit Destillationsbrücke und Impellerrührer wurden bei 120°C in die Schmelze von 630 g (4,0 mol) 2-Chlornitrobenzol 290,5 g (5,0 mol) Kaliumfluorid, 71,1 g (0,1 mol) Trimethyl(ethoxypolyoxypropyl)-ammoniumchlorid, 11,0 g (0,1 mol) Tetramethylammoniumchlorid und 17,7 g (0,035 mol) Polyethylenglykoldimethylether 500 eingetragen. Anschließend wurde mit 100 g (0,94 mol) Xylol azeotrop getrocknet und die Reaktionsmischung 28 Stunden bei einer Temperatur von 150°C gerührt.
Entstandene Menge an 2-Fluornitrobenzol:
- nach 6 Stunden:: 36 GC-Flächen-%
- nach 28 Stunden:: 74 GC-Flächen-%.

### Beispiel 3: 2,3,4-Trifluornitrobenzol

In einen 1,5-Liter Planschliffkolben mit Destillationsbrücke und Ankerrührer wurden bei 110°C in die Schmelze von 840 g (4 mol) 2,4-Dichlor-3-fluornitrobenzol 488 g (8,4 mol) Kaliumfluorid und 40,0 g (0,07 mol) Dimethyl-di-(ethoxypolyoxypropylmethylether)-ammoniumchlorid, 20,0 g (0,04 mol) Polyethylenglykoldimethylether 500 und 20,4 g (0,06 mol) Tetrabutylphosphoniumbromid eingetragen. Anschließend wurde mit 60 g (0,57 mol) Xylol azeotrop getrocknet und 21 Stunden bei einer Temperatur von 150°C gerührt.
Entstandene Menge an 2,3,4-Trifluornitrobenzol:
- nach 5 Stunden:: 13 GC-Flächen-%
- nach 21 Stunden:: 65 GC-Flächen-%.

### Beispiel 4: 2,4-Dichlor-5-fluorbenzonitril

In einen 250 ml Dreihalskolben mit Rückflußkühler und Blattrührer wurden 97,6 g (0,4 mol) 5-Brom-2,4-dichlorfluorbenzol, 35,8 g (0,4 mol) Kupfer(I)cyanid, 10,0 g (0,014 mol) Trimethyl-(ethoxypolyoxypropyl)ammoniumchlorid, 5,0 g (0,015 mol) Tetrabutylphosphoniumbromid und 5,0 g (0,01 mol) Polyethylenglykoldimethylether 500 zusammen mit 20,0 g (0,19 mol) Xylol eingetragen und bis 150°C unter vermindertem Druck azeotrop getrocknet.
Entstandene Menge an 2,4-Dichlor-5-fluorbenzonitril:
- nach 5 Stunden:: 19 GC-Flächen-%
- nach 26 Stunden:: 75 GC-Flächen-%.

### Vergleichsbeispiel 1: 2,4-Dichlor-5-fluorbenzonitril

In einen 100 ml Dreihalskolben mit Rückflußkühler und Blattrührer wurden 48,8 g (0,2 mol) 5-Brom-2,4-dichlorfluorbenzol und 17,9 g (0,2 mol) Kupfer(I)cyanid eingetragen und bei 150°C für 4 Stunden gerührt (keine Reaktion). Die Reaktionssuspension wurde auf 200°C erhitzt und weitere 4 Stunden bei dieser Temperatur gerührt: keine Umsetzung, 0 GC-Flächen-%.

### Beispiel 5: 3-Chlor-2-fluornitrobenzol

In einen 1,5-Liter Planschliffkolben mit Destillationsbrücke und Impellerrührer wurden bei 100°C in die Schmelze von 768 g (4 mol) 2,3-Dichlornitrobenzol 279 g (4,8 mol) Kaliumfluorid, 218,5 g (0,3 mol) Trimethyl(ethoxypolyoxypropyl)-ammoniumchlorid und 11,5 g (0,023 mol) Polyethylenglykoldimethylether 500 eingetragen. Anschließend wurde mit 200 g (1,9 mol) Xylol azeotrop getrocknet und 24 Stunden bei einer Temperatur von 100°C gerührt.
Entstandene Menge an 3-Chlor-2-fluornitrobenzol:
- nach 5 Stunden:: 18 GC-Flächen-%
- nach 24 Stunden:: 47 GC-Flächen-%.

### Vergleichsbeispiel 2: 3-Chlor-2-fluornitrobenzol

In einen 100 ml Planschliffkolben mit Rückflußkühler und Blattrührer wurden 57,6 g (0,3 mol) 2,3-Dichlornitrobenzol und 13,9 g (0,24 mol) Kaliumfluorid eingetragen und bei 150°C 2 Stunden gerührt (keine Reaktion). Die Reaktionssuspension wurde auf 190°C erhitzt und weitere 4 Stunden bei dieser Temperatur gerührt.
Entstandene Menge an 3-Chlor-2-fluornitrobenzol:
- nach 2 Stunden:: 1 GC-Flächen-%
- nach 8 Stunden:: 8 GC-Flächen-%.

### Beispiel 6: 4-Fluorbenzonitril

In einen 250 ml Planschliffkolben mit Impeller-Rührer wurden bei 120°C in die Schmelze von 137,6 g (1,0 mol) 4-Chlorbenzonitril 69,6 g (1,2 mol) Kaliumfluorid, 6,9 g (0,02 mol) Tetrabutylphosphoniumbromid, 13,8 g (0,02 mol) Dimethyl-di(ethoxypolyoxypropyl)-ammoniumchlorid, 13,8 g (0,028 mol) Polyethylenglykoldimethylether 500 und 20 g Xylol zudosiert. Nach 30 Minuten Rühren wurde die Reaktionssuspension durch Abdestillieren des Xylols unter Vakuum azeotrop getrocknet. Die Reaktionssuspension wurde 30 Stunden bei 190°C gerührt.
Entstandene Menge an 4-Fluorbenzonitril:
- nach 9 Stunden:: 12 GC-Flächen-%
- nach 30 Stunden:: 48 GC-Flächen-%, neben 45 GC-Flächen-% nicht umgesetztem 4-Chlorbenzonitril.

### Beispiel 7: 5-Chlor-2-nitrophenol

57,6 g (0,3 mol) 2,4-Dichlornitrobenzol wurden mit 10 g (0,014 mol) Dimethyl-di(ethoxypolyoxypropyl)-ammoniumchlorid und 150 ml 20 Gew.-%iger NaOH bei 60°C versetzt. Das zweiphasige Reaktionsgemisch wurde 21 Stunden bei 100°C gerührt und anschließend mit HCl auf pH 1 gebracht.
Entstandene Menge an 5-Chlor-2-nitrophenol.
- nach 21 Stunden:: 40 GC-Flächen-%.

## Patentansprüche

1. Katalysator für nukleophile Substitutionen, bestehend im wesentlichen aus einer Mischung aus
a) einer oder mehreren quartären Ammoniumverbindung(en) der Formel (1) 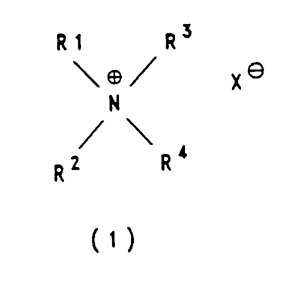 worin
R¹, R² und R³ gleich oder verschieden sind und
einen linearen oder verzweigten Rest der Formel -(CₘH₂ₘO)ₚR⁵ bedeuten, worin R⁵ für Wasserstoff oder einen linearen oder verzweigten Alkylrest mit 1 bis 16 Kohlenstoffatomen steht, m eine ganze Zahl von 1 bis 10 und p eine Zahl von 1 bis 15 bedeuten;
oder einen linearen oder verzweigten Alkylrest mit 1 bis 30 Kohlenstoffatomen;
oder einen unsubstituierten Phenyl- oder Naphthylrest; oder einen substituierten Phenyl- oder Naphthylrest, wobei die Substituenten die Bedeutung Halogen, C₁-C₄-Alkyl, C₁-C₄-Alkoxy, Nitro oder Cyano haben;
R⁴ einen linearen oder verzweigten Rest der Formel -(CₘH₂ₘO)ₚR⁵ bedeutet; und
X^{⊖} ein anorganisches Anion ist;
und
b) einem oder mehreren quartären Ammoniumsalz(en) oder Phosphoniumsalz(en) der Formel (2) 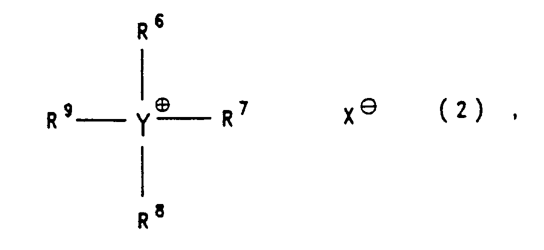 worin
R⁶, R⁷, R⁸ und R⁹ gleich oder verschieden sind und
einen linearen oder verzweigten Alkylrest mit 1 bis 22 Kohlenstoffatomen; oder einen unsubstituierten oder substituierten Arylrest oder einen C₁-C₄-Alkyl-arylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten; und
Y die Bedeutung N oder P hat;
oder aus einer Mischung aus einer oder mehreren Verbindung(en) der Formel (1) und
c] einem oder mehreren Ether(n) der Formel (3)
R¹⁰-(O-CₓH₂ₓ)ᵣ-OR¹¹ (3),
worin
R¹⁰ und R¹¹ gleich oder verschieden sind und Wasserstoff oder einen linearen oder verzweigten Alkylrest mit 1 bis 16 Kohlenstoffatomen bedeuten;
x eine ganze Zahl von 2 bis 6 und
r eine Zahl von 0 bis 20 ist;
oder einem Kronenether;
oder aus einer Mischung der in a), b) und c) genannten Verbindungen.

2. Katalysator nach Anspruch 1, dadurch gekennzeichnet, daß
R¹, R² und R³ gleich oder verschieden sind und einen linearen oder verzweigten Rest der Formel -(CₘH₂ₘO)ₚR⁵ bedeuten, worin R⁵ für Wasserstoff oder einen linearen oder verzweigten Alkylrest mit 1 bis 8 Kohlenstoffatomen steht, m eine ganze Zahl von 1 bis 5 und p eine Zahl von 2 bis 10 bedeuten; oder einen linearen oder verzweigten Alkylrest mit 1 bis 18 Kohlenstoffatomen; oder einen unsubstituierten Phenyl- oder Naphthylrest, oder einen substituierten Phenyl- oder Naphthylrest, wobei die Substituenten die Bedeutung Halogen, C₁-C₄-Alkyl, C₁-C₄-Alkoxy, Nitro oder Cyano haben;
R⁴ einen linearen oder verzweigten Rest der Formel -(CₘH₂ₘO)ₚR⁵ bedeutet, worin R⁵ für Wasserstoff oder einen linearen oder verzweigten Alkylrest mit 1 bis 8 Kohlenstoffatomen steht, m eine ganze Zahl von 1 bis 5 und p eine Zahl von 2 bis 10 bedeuten; und
X^{⊖} Fluorid, Chlorid, Bromid, SO₄²⁻/2 oder Hydrogensulfat ist.

3. Katalysator nach Anspruch 1, dadurch gekennzeichnet, daß
R⁶, R⁷, R⁸ und R⁹ gleich oder verschieden sind und einen linearen oder verzweigten Alkylrest mit 1 bis 16 Kohlenstoffatomen; oder einen unsubstituierten oder substituierten Arylrest oder einen C₁-C₄-Alkylarylrest, wobei Aryl die Bedeutung Phenyl oder Naphthyl hat und die besagten Substituenten Halogen, C₁-C₄-Alkyl, C₁-C₄-Alkoxy, Nitro oder Cyano bedeuten.

4. Katalysator nach Anspruch 1, dadurch gekennzeichnet, daß
R¹⁰ und R¹¹ gleich oder verschieden sind und Wasserstoff oder einen linearen oder verzweigten Alkylrest mit 1 bis 8 Kohlenstoffatomen,
x eine ganze Zahl von 2 bis 3 und
r eine Zahl von 4 bis 14 bedeuten.

5. Katalysator nach Anspruch 1 oder 2, dadurch gekennzeichnet, daß die Komponente a) 5 bis 95 Gew.-% des gesamten Katalysators ausmacht.

6. Katalysator nach Anspruch 1 oder 2, dadurch gekennzeichnet, daß die Komponente a) 20 bis 80 Gew.-% des gesamten Katalysators ausmacht.

7. Katalysator nach Anspruch 1 oder 2, dadurch gekennzeichnet, daß in der Verbindung der Formel (1) ein oder zwei Reste der Formel -(CₘH₂ₘO)ₚR⁵ enthalten sind.

8. Katalysator nach mindestens einem der Ansprüche 1 bis 7, dadurch gekennzeichnet, daß die Verbindung der Formel (1) Dimethyl-di(ethoxypolyoxypropyl)-ammoniumchlorid, Dimethyl-di(ethoxypolyoxypropylmethylether)-ammoniumchlorid, Dimethyl-(ethoxypolyoxypropyl)-(ethoxypolyoxypropylmethylether)-ammoniumchlorid, Dimethyl-di(ethoxypolyoxyethyl)-ammoniumchlorid, Dimethyl-di(ethoxypolyoxyethylmethylether)-ammoniumchlorid, Dimethyl(ethoxypolyoxyethyl)-(ethoxypolyoxyethylmethylether)-ammoniumchlorid, jeweils mit einer mittleren Kettenlänge p von 3, oder Trimethyl(ethoxypolyoxypropyl)-ammoniumchlorid oder Trimethyl(ethoxypolyoxypropylmethylether)-ammoniumchlorid, jeweils mit einer mittleren Kettenlänge p von 8, oder ein Gemisch der genannten Verbindungen ist.

9. Katalysator nach mindestens einem der Ansprüche 1 bis 8, dadurch gekennzeichnet, daß die Verbindung der Formel (2) Octadecyltrimethylammoniumchlorid, Distearyldimethylammoniumchlorid, Tetramethylammoniumchlorid, Tetramethylammoniumbromid, Hexadecyltrimethylammoniumchlorid, Benzyltrimethylammoniumchlorid, Hexadecyltributylphosphoniumbromid, Stearyltributylphosphoniumbromid, Tetrabutylphosphoniumchlorid, Tetrabutylphosphoniumbromid oder Tetraoctylphosphoniumbromid ist.

10. Katalysator nach mindestens einem der Ansprüche 1 bis 9, dadurch gekennzeichnet, daß die Komponente c) eine Mischung von Polyethylenglykoldimethylethern ist und eine mittlere Molmasse von 500 hat.

## Claims

1. A catalyst for nucleophilic substitutions, consisting essentially of a mixture of
a) one or more quaternary ammonium compound(s) of the formula (1) 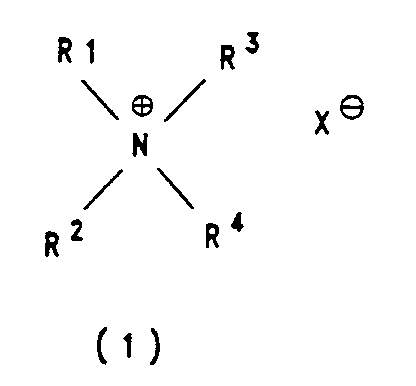 in which
R¹, R² and R³ are identical or different and
are a linear or branched radical of the formula -(CₘH₂ₘO) ₚR⁵, in which R⁵ is hydrogen or a linear or branched alkyl radical having from 1 to 16 carbon atoms, m is an integer from 1 to 10 and p is a number from 1 to 15; or a linear or branched alkyl radical having from 1 to 30 carbon atoms;
or an unsubstituted phenyl or naphthyl radical; or a substituted phenyl or naphthyl radical, with the substituents being halogen, C₁-C₄-alkyl, C₁-C₄-alkoxy, nitro or cyano;
R⁴ is a linear or branched radical of the formula -(CₘH₂ₘO)ₚR⁵; and
X⁻ is an inorganic anion;
and
b) one or more quaternary ammonium salt(s) or phosphonium salt(s) of the formula (2) 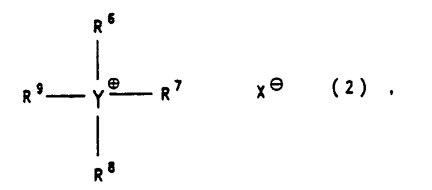 in which
R⁶, R⁷, R⁸ and R⁹ are identical or different and are a linear or branched alkyl radical having from 1 to 22 carbon atoms; or an unsubstituted or substituted aryl radical or a C₁-C₄-alkyl-aryl radical, with aryl being phenyl or naphthyl and the said substituents being halogen, C₁-C₄-alkyl, C₁-C₄-alkoxy, nitro or cyano; and
Y is N or P;
or of a mixture of one or more compound(s) of the formula (1)
and
c) one or more ether(s) of the formula (3)
R¹⁰-(O-Cₓ-H₂ₓ)ᵣ-OR¹¹ (3)
, in which
R¹⁰ and R¹¹ are identical or different and are hydrogen or a linear or branched alkyl radical having from 1 to 16 carbon atoms;
x is an integer from 2 to 6 and
r is a number from 0 to 20;
or a crown ether;
or of a mixture of the compounds specified in a), b) and c).

2. A catalyst as claimed in claim 1, wherein
R¹, R² and R³ are identical or different and are a linear or branched radical of the formula -(CₘH₂ₘO)ₚR⁵, in which R⁵ is hydrogen or a linear or branched alkyl radical having from 1 to 8 carbon atoms, m is an integer from 1 to 5 and p is a number from 2 to 10; or a linear or branched alkyl radical having from 1 to 18 carbon atoms; or an unsubstituted phenyl or naphthyl radical, or a substituted phenyl or naphthyl radical, with the substituents being halogen, C₁-C₄-alkyl, C₁-C₄-alkoxy, nitro or cyano;
R⁴ is a linear or branched radical of the formula -(CₘH₂ₘO)ₚR⁵, in which R⁵ is hydrogen or a linear or branched alkyl radical having from 1 to 8 carbon atoms, m is an integer from 1 to 5 and p is a number from 2 to 10; and
X⁻ is fluoride, chloride, bromide, SO₄²⁻/2 or hydrogen sulfate.

3. A catalyst as claimed in claim 1, wherein
R⁶, R⁷, R⁸ and R⁹ are identical or different and are a linear or branched alkyl radical having from 1 to 16 carbon atoms; or an unsubstituted or substituted aryl radical or a C₁-C₄-alkyl-aryl radical, with aryl being phenyl or naphthyl and the said substituents being halogen, C₁-C₄-alkyl, C₁-C₄-alkoxy, nitro or cyano.

4. A catalyst as claimed in claim 1, wherein
R¹⁰ and R¹¹ are identical or different and are hydrogen or a linear or branched alkyl radical having from 1 to 8 carbon atoms,
x is an integer from 2 to 3 and
r is a number from 4 to 14.

5. A catalyst as claimed in claim 1 or 2, wherein the component a) makes up from 5 to 95% by weight of the total catalyst.

6. A catalyst as claimed in claim 1 or 2, wherein the component a) makes up from 20 to 80% by weight of the total catalyst.

7. A catalyst as claimed in claim 1 or 2, wherein one or two radicals of the formula -(CₘH₂ₘO)ₚR⁵ are present in the compound of the formula (1).

8. A catalyst as claimed in at least one of claims 1 to 7, wherein the compound of the formula (1) is dimethyldi(ethoxypolyoxypropyl)ammonium chloride, dimethyldi(ethoxypolyoxypropyl methyl ether)ammonium chloride, dimethyl(ethoxypolyoxypropyl)(ethoxypolyoxypropyl methyl ether)ammonium chloride, dimethyldi(ethoxypolyoxyethyl)ammonium chloride, dimethyldi(ethoxypolyoxyethyl methyl ether)ammonium chloride, dimethyl(ethoxypolyoxyethyl)(ethoxypolyoxyethyl methyl ether)ammonium chloride, each having a mean chain length p of 3, or trimethyl(ethoxypolyoxypropyl)ammonium chloride or trimethyl(ethoxypolyoxypropyl methyl ether)ammonium chloride, each having a mean chain length p of 8, or a mixture of the specified compounds.

9. A catalyst as claimed in at least one of claims 1 to 8, wherein the compound of the formula (2) is octadecyltrimethylammonium chloride, distearyldimethylammonium chloride, tetramethylammonium chloride, tetramethylammonium bromide, hexadecyltrimethylammonium chloride, benzyltrimethylammonium chloride, hexadecyltributylphosphonium bromide, stearyltributylphosphonium bromide, tetrabutylphosphonium chloride, tetrabutylphosphonium bromide or tetraoctylphosphonium bromide.

10. A catalyst as claimed in at least one of claims 1 to 9, wherein the component c) is a mixture of polyethylene glycol dimethyl ethers and has a mean molecular mass of 500.

## Revendications

1. Catalyseur pour substitutions nucléophiles constitué essentiellement d'un mélange
a) d'un ou plusieurs composé(s) d'ammonium quaternaire de formule (1) 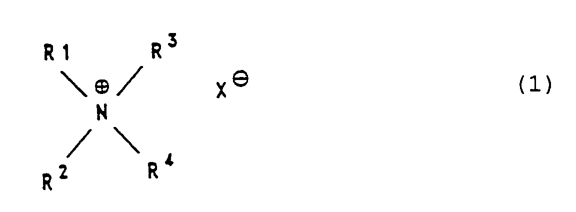 dans laquelle
R¹, R² et R³ sont identiques ou différents et représentent un reste linéaire ou ramifié de formule -(CₘH₂ₘO)ₚR⁵, dans laquelle R⁵ représente un atome d'hydrogène ou un reste alkyle linéaire ou ramifié avec de 1 à 16 atomes de carbone, m est un entier de 1 à 10 et p est un noire de 1 à 15 ; ou un reste alkyle linéaire ou ramifié avec de 1 à 30 atomes de carbone ;
ou un reste phényle ou naphtyle non substitué ; ou un reste phényle ou naphtyle substitué, les substituants représentant un atome d'halogène, des groupes alkyle en C₁-C₄, alkoxy en C₁-C₄, nitro ou cyano ;
R⁴ représente un reste linéaire ou ramifié de formule -(CₘH₂ₘO)ₚR⁵ ; et
X⁻ représente un anion inorganique ;
et
b) d'un ou plusieurs sel(s) d'ammonium ou de phosphonium quaternaire de formule (2) 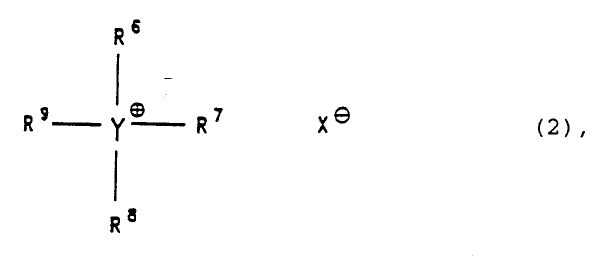 dans laquelle
R⁶, R⁷, R⁸ et R⁹ sont identiques ou différents et représentent un reste alkyle linéaire ou ramifié avec de 1 à 22 atomes de carbone ; ou un reste aryle non substitué ou substitué ou un reste (alkyl en C₁-C₄)aryle, le reste aryle ayant la signification d'un groupe phényle ou naphtyle et lesdits substituants représentent un atome d'halogène, des groupes alkyle en C₁-C₄, alkoxy en C₁-C₄, nitro ou cyano ; et
Y représente N ou P ;
ou d'un mélange d'un ou plusieurs composés de formule (1)
et
c) d'un ou plusieurs éther(s) de formule (3)
R¹⁰-(O-CₓH₂ₓ)ᵣ-OR¹¹ (3),
dans laquelle
R¹⁰ et R¹¹ sont identiques ou différents et représentent un atome d'hydrogène ou un reste alkyle linéaire ou ramifié avec de 1 à 16 atomes de carbone ;
x représente un entier de 2 à 6 et
r représente un nombre de 0 à 20 ;
ou un éther-couronne ;
ou d'un mélange des composés cités aux points a), b) et c).

2. Catalyseur selon la revendication 1, caractérisé en ce que
R¹, R² et R³ sont identiques ou différents et représentent un reste linéaire ou ramifié de formule -(CₘH₂ₘO)ₚR⁵, dans laquelle R⁵ représente un atome d'hydrogène ou un reste alkyle linéaire ou ramifié avec de 1 à 8 atomes de carbone, m représente un entier de 1 à 5 et p est un nombre de 2 à 10 ; ou un reste alkyle linéaire ou ramifié avec de 1 à 18 atomes de carbone ; ou un reste phényle ou naphtyle non substitué ; ou un reste phényle ou naphtyle substitué, les substituants ayant les significations d'un atome d'halogène, des groupes alkyle en C₁-C₄, alkoxy en C₁-C₄, nitro ou cyano ;
R⁴ représente un reste linéaire ou ramifié de formule -(CₘH₂ₘO)ₚR⁵ , dans laquelle R⁵ représente un atome d'hydrogène ou un reste alkyle linéaire ou ramifié avec de 1 à 8 atomes de carbone, m est un entier de 1 à 5 et p un nombre de 2 à 10 ; et
X⁻ représente fluorure, chlorure, bromure, SO₄²⁻/2 ou bisulfate.

3. Catalyseur selon la revendication 1, caractérisé en ce que
R⁶, R⁷, R⁸ et R⁹ sont identiques ou différents et représentent un reste alkyle linéaire ou ramifié avec de 1 à 16 atomes de carbone ; ou un reste aryle ou un reste (alkyl en C₁-C₄)aryle substitué ou non substitué, aryle ayant la signification d'un groupe phényle ou naphtyle et lesdits substituants représentant un atome d'halogène, des groupes alkyle en C₁-C₄, alkoxy en C₁-C₄, nitro ou cyano.

4. Catalyseur selon la revendication 1, caractérisé en ce que
R¹⁰ et R¹¹ sont identiques ou différents et représentent un atome d'hydrogène ou un reste alkyle linéaire ou ramifié avec de 1 à 8 atomes de carbone ;
x représente un entier de 2 à 3, et
r représente un nombre de 4 à 14.

5. Catalyseur selon la revendication 1 ou 2, caractérisé en ce que le composant a) représente de 5 à 95 % en poids par rapport à la totalité du catalyseur.

6. Catalyseur selon la revendication 1 ou 2, caractérisé en ce que le composant a) représente de 20 à 80 % en poids de la totalité du catalyseur.

7. Catalyseur selon la revendication 1 ou 2, caractérisé en ce que le composé de formule (1) contient un ou deux restes de formule -(CₘH₂ₘO)ₚR⁵.

8. Catalyseur selon au moins l'une des revendications 1 à 7, caractérisé en ce que le composé de formule (1) est le chlorure de diméthyl-di(éthoxypolyoxypropyl)-ammonium, le chlorure de diméthyldi(éthoxypolyoxypropylméthyléther)-ammonium, le chlorure de diméthyl-(éthoxypolyoxypropyl)-(éthoxypolyoxypropylméthyléther)-ammonium, le chlorure de diméthyl-di(éthoxypolyoxyéthyl)-ammonium, le chlorure de diméthyldi(éthoxypolyoxyéthylméthyléther)-ammonium, le chlorure de diméthyl-(éthoxypolyoxyéthyl)-(éthoxypolyoxyéthylméthyléther)-ammonium, ayant à chaque fois une longueur moyenne de chaîne p égale à 3, ou le chlorure de triméthyl(éthoxypolyoxypropyl)-ammonium ou le chlorure de triméthyl-(éthoxypolyoxypropylméthyléther)-ammonium, ayant une longueur moyenne de chaîne p égale à 8, ou un mélange des composés précités.

9. Catalyseur selon au moins l'une des revendications 1 à 8, caractérisé en ce que le composé de formule (2) est le chlorure d'octadécyltriméthylammonium, le chlorure de distéaryldiméthylammonium, le chlorure de tétraméthylammonium, le bromure de tétraméthylammonium, le chlorure d'hexadécyltriméthylammonium, le chlorure de benzyltriméthylammonium, le bromure d'hexadécyltributylphosphonium, le bromure de stéaryltributylphosphonium, le chlorure de tétrabutylphosphonium, le bromure de tétrabutylphosphonium ou le bromure de tétraoctylphosphonium.

10. Catalyseur selon au moins l'une des revendications 1 à 9, caractérisé en ce que le composant c) est un mélange de diméthyléthers de polyéthylèneglycol et présente une masse molaire moyenne de 500.
